# EUROPEAN PATENT APPLICATION

(11) **EP 4 801 237 A1**
(43) Date of publication of application: **02.09.2026**
(21) Application number: 24882500.2
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086009
(71) Applicant: Canon Kabushiki Kaisha, Tokyo 146-8501 (JP)
(72) Inventor: NAKAMURA Nobuhiro, Tokyo 146-8501 (JP); OHSAWA Tatsuya, Tokyo 146-8501 (JP); SEKIDO Kunihiko, Tokyo 146-8501 (JP); NISHIDA Tsutomu, Tokyo 146-8501 (JP)
(74) Representative: TBK
(86) International application number: PCT/JP2024/038127
(87) International publication number: WO 2025/089392

(57) **Abstract**

Provided are a photoelectric conversion element and a photoelectric conversion apparatus each of which achieves both of a high open circuit voltage and high durability. The photoelectric conversion element includes: a first electrode; a second electrode; and a photoelectric conversion layer arranged between the first electrode and the second electrode, the photoelectric conversion layer containing a crystal having a perovskite structure. The photoelectric conversion element is characterized by further including, between the photoelectric conversion layer and the first electrode, a charge-transporting layer containing: a crystal of a cyclic conjugated compound in which pyrrole rings are bonded by conjugated bonds, the compound having an axial ligand; and a resin having a Lewis basic functional group.

## Description

### [Technical Field]

The present invention relates to a photoelectric conversion element and a photoelectric conversion apparatus.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage utilizing a photovoltaic effect in which light energy is absorbed from sunlight to generate an electron and a hole.

Currently, an n-p diode-type silicon (Si) single crystal-based solar cell having a light energy conversion efficiency of more than 20% is widely known, and is actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate, and hence a cost reduction can be expected. However, further improvements in conversion efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion property.

For example, in Patent Literature 1, there is a description of the following technology: the peeling of a hole-transporting layer (hereinafter, also referred to as "charge-transporting layer") from an anode is suppressed by mixing an insulating polymer and a hole-transporting material thereinto, and hence conversion efficiency and durability are improved. In Non Patent Literature 1, there is a description of a technology including mixing copper phthalocyanine and a conductive polymer into a hole-transporting layer to improve conversion efficiency.

### [Citation List]

### [Patent Literature]

PTL 1: Japanese Patent Laid-Open No. 2018-170382

### [Non Patent Literature]

NPL 1: Q. Hu, et al, Sol. RRL, 2019, 3, 1800264

### [Summary of Invention]

### [Technical Problem]

According to investigations made by the inventors of the present invention, each of the photoelectric conversion elements of Patent Literature 1 and Non Patent Literature 1 has had a problem in achieving both of a high open circuit voltage (Voc) and high durability. Accordingly, the present invention is directed to providing a photoelectric conversion element and a photoelectric conversion apparatus each of which achieves both of a high open circuit voltage and high durability.

### [Solution to Problem]

The above-mentioned provision of the element and the apparatus is achieved by the present invention described below. That is, a photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure, wherein the photoelectric conversion element further includes, between the photoelectric conversion layer and the first electrode, a charge-transporting layer containing: a crystal of a cyclic conjugated compound in which pyrrole rings are bonded by conjugated bonds, the compound having an axial ligand; and a resin having a Lewis basic functional group.

### [Advantageous Effects of Invention]

According to the present invention, the photoelectric conversion element that achieves both of a high open circuit voltage and high durability can be provided.

### [Brief Description of Drawings]

[Fig. 1]

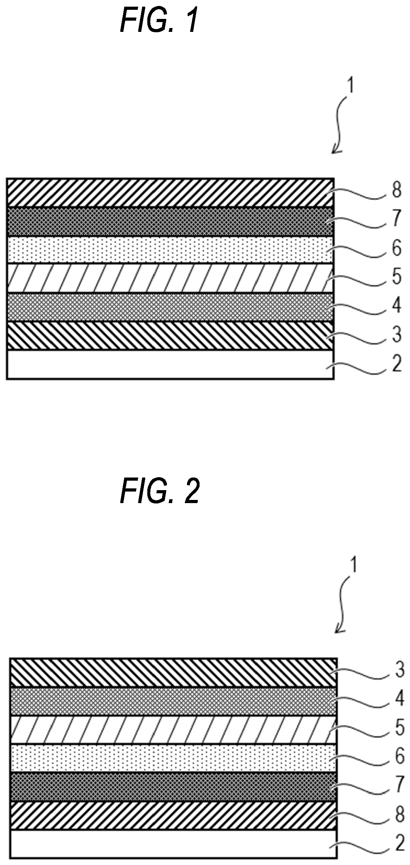

   Fig. 1 is an example of a schematic sectional view in the thickness direction of a photoelectric conversion element according to one embodiment of the present invention.
[Fig. 2]
   Fig. 2 is another example of a schematic sectional view in the thickness direction of the photoelectric conversion element according to one embodiment of the present invention.
[Fig. 3]

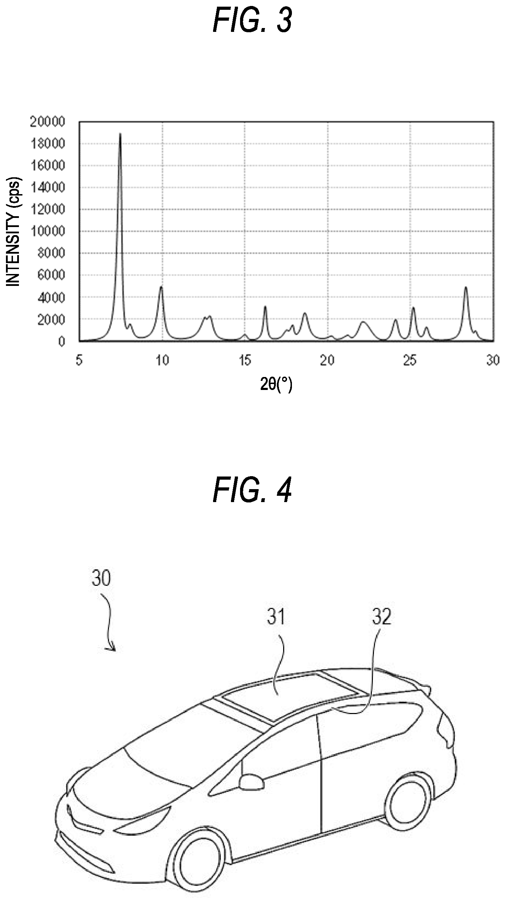

   Fig. 3 is an X-ray diffraction spectrum using CuKα rays of a crystal of a cyclic conjugated compound in which pyrrole rings are bonded by conjugated bonds, the compound having an axial ligand, of the present invention.
[Fig. 4]
   Fig. 4 is a perspective view for schematically illustrating a moving body including the photoelectric conversion element according to one embodiment of the present invention.
[Fig. 5]

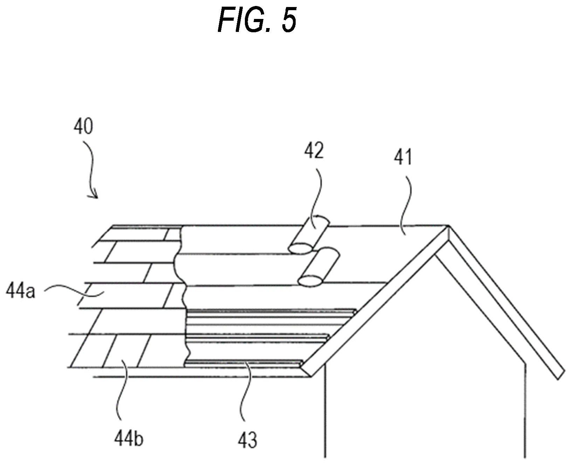

   Fig. 5 is a perspective view for schematically illustrating a building material including the photoelectric conversion element according to one embodiment of the present invention.

### [Description of Embodiments]

A photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure, the photoelectric conversion element further including, between the photoelectric conversion layer and the first electrode, a charge-transporting layer containing: a crystal of a cyclic conjugated compound in which pyrrole rings are bonded by conjugated bonds, the compound having an axial ligand; and a resin having a Lewis basic functional group.

As a result of investigations, the inventors of the present invention have found that, when a photoelectric conversion element includes the above-mentioned charge-transporting layer, the photoelectric conversion element has a high open circuit voltage and excellent durability. Although the details are not clear, a possible reason why the photoelectric conversion element having a high open circuit voltage and excellent durability is obtained in the present invention is as described below.

The cyclic conjugated compound in which pyrrole rings are bonded by covalent bonds has a planar structure in which a π-electron conjugated system spreads over the entire molecule. When a film using the cyclic conjugated compound as a charge-transporting material is formed, high hole-transporting properties are exhibited. In addition, the cyclic conjugated compound can form complexes with various elements in the center of the ring, and can take an axial ligand so as to be positioned in the vertical direction of the plane of the cyclic conjugated compound.

A photoelectric conversion element exhibiting a high hole-transporting ability may be produced by forming a film of, for example, a crystal of a phthalocyanine compound having an axial ligand as the cyclic conjugated compound in which pyrrole rings are bonded by covalent bonds between the photoelectric conversion layer and the electrode. At this time, the use of the crystal of the phthalocyanine compound having an axial ligand is more advantageous for durability than the use of a crystal of a phthalocyanine compound having no axial ligand is. A possible reason for the foregoing is as described below. In a perovskite solar cell, there is a phenomenon in which the structure of the photoelectric conversion element is broken by the migration of water molecules from the outside, or ions, molecules, or the like for forming the photoelectric conversion element to other layers. Meanwhile, a presumed reason for the foregoing is that the progress of the migration is suppressed by trapping the migrated water molecules, ions, molecules, or the like in a space inside the phthalocyanine crystal having an axial ligand. In the case of a phthalocyanine crystal having no axial ligand, it is difficult to trap the molecules or ions in the space because of a structure in which planar structures are stacked. However, in the case of a phthalocyanine crystal having an axial ligand, the molecules or ions may be easily trapped in the space because of a crystal structure formed by the repetition of a structure in which the axial ligand protrudes from the planar structure.

However, when the phthalocyanine crystal having an axial ligand was used, a reduction in open circuit voltage of the photoelectric conversion element was observed as compared to the case of the phthalocyanine crystal having no axial ligand. A possible reason for the foregoing is as described below. The phthalocyanine having an axial ligand has a large moment in which charge is biased because the axial ligand protrudes from the planar structure. This is presumed to be because when the photoelectric conversion element is irradiated with light and a voltage is generated, the large bias of the charge acts so as to cancel the generated voltage. Thus, there has been a problem in achieving a photoelectric conversion element having a high open circuit voltage and durability for maintaining the open circuit voltage.

In the present invention, it has been found that a high open circuit voltage and high durability can be achieved by incorporating, into a charge-transporting layer, a crystal of phthalocyanine having an axial ligand, which is a cyclic conjugated compound in which pyrrole rings are bonded by covalent bonds, and a resin having a Lewis basic functional group. This is probably because the large moment of the phthalocyanine crystal having an axial ligand is reduced by the electronic action of the Lewis basic functional group on the phthalocyanine crystal having an axial ligand. It is presumed that a photoelectric conversion element that achieves both of durability and a high voltage can be achieved by optimizing the moment while maintaining the crystal structure effective for durability.

The cyclic conjugated compound of the present invention in which pyrrole rings are bonded by covalent bonds has a central element for having an axial ligand. Various elements may each be adopted as the central element, but Ga, Ti, V, Al, In, Fe, and Mn are preferred because of their high charge-transporting abilities. The cyclic conjugated compound more preferably has at least one central element selected from the group consisting of: Ga; Ti; V; Al; In; Fe; and Mn. Of those, Ga is still more preferred from the viewpoint of an electronic interaction with the resin having a Lewis basic functional group.

The axial ligand may come in various kinds, such as a halogen atom, an alkyl group, an aryl group, a carboxy group, an alkoxy group, a hydroxy group, a cyano group, an amino group, and an oxygen atom. Of those, OH, Cl, and O are each preferred from the viewpoint of an electronic interaction with the resin having a Lewis basic functional group. The axial ligand is preferably at least one selected from the group consisting of: OH; Cl; and O.

The crystal of the cyclic conjugated compound of the present invention in which pyrrole rings are bonded by conjugated bonds has an axial ligand, and hence the crystal has a large moment and a high charge-transporting ability. The presence of an axial ligand on one side of the planar structure generates a large moment. When two axial ligands are present, a moment is generated also in the case where two ligands coordinate from one side of the planar structure or in the case where different ligands are present on both the sides of the planar structure. Thus, the ligands interact with the Lewis basic functional group and the effect of the present invention is exhibited. The number of the axial ligands of the cyclic conjugated compound is preferably one because an interaction with the Lewis basic functional group easily occurs.

A cyclic conjugated compound having crystallinity is used as the cyclic conjugated compound of the present invention in which pyrrole rings are bonded by conjugated bonds. In the present invention, it can be recognized by an X-ray diffraction spectrum described below that the planar structure of the cyclic conjugated compound in which pyrrole rings are bonded by conjugated bonds has crystal structures regularly arranged in at least two directions. The crystallinity may facilitate the trapping of water molecules and ions, and lead to the exhibition of an effect on durability. The foregoing can be recognized by the full width at half maximum of a peak of an X-ray diffraction spectrum using CuKα rays for the compound. A smaller full width at half maximum means that a crystallite becomes larger and its crystallinity becomes stronger. In the present invention, the full width at half maximum of each of the maximum peak (first peak) and the next largest peak (second peak) in the range where 2θ is 5 to 30° in the X-ray diffraction spectrum only needs to be 1.00° or less. For example, when a large peak is present in the range where 2θ is 5 to 10°, many crystals may be stacked in a direction in which the planar structures of the cyclic conjugated compound in which pyrrole rings are bonded by conjugated bonds overlap. In addition, when a large peak is present in the range where 20 is 25 to 30°, many crystals may be stacked in a direction in which the planar structures of the cyclic conjugated compound in which pyrrole rings are bonded by conjugated bonds are arranged laterally. For example, in Fig. 3, the Bragg angle 20 at which a first peak appears is 7.5°, and its full width at half maximum is 0.30°. In addition, the Bragg angle 20 at which a second peak appears is 28.3°, and its full width at half maximum is 0.35°. Accordingly, the compound shown in Fig. 3 is a compound having the crystallinity of the present invention.

Specifically, the compound is packed in a Boro-Silicate capillary (length: 70 nm, wall thickness: 0.01 mm, inner diameter: 0.7 mm) (manufactured by W. Muller), and is subjected to X-ray diffraction measurement under the following conditions.
Measurement instrument used: X-ray diffractometer RINT-TTRII manufactured by Rigaku Corporation
X-ray tube: Cu
X-ray wavelength: Kα1
Tube voltage: 50 kV
Tube current: 300 mA
Scan method: 20-0 scan
Scan speed: 4.0°/min
Sampling interval: 0.02°
Start angle 2θ: 5.0°
Stop angle 2θ: 30.0°
Goniometer: Rotor horizontal goniometer (TTR-2)
Attachment: capillary rotating sample stage
Filter: none
Detector: scintillation counter
Incident monochromator: used
Slit: variable slit (parallel beam)
Counter monochromator: not used
Divergence slit: open
Divergence longitudinal limiting slit: 10.00 mm
Scattering slit: open
Receiving slit: open

After that, appropriate processing, such as smoothing processing, background removal, or Kα2 removal, is performed on the obtained data, and then fitting is performed by a profile function to provide an X-ray diffraction spectrum. Specifically, integrated powder X-ray analysis software PDXL is used, manual processing is selected in data processing, and the X-ray diffraction spectrum is obtained under the following conditions.
Smoothing: smoothing by B-spline, x threshold: 1.50
Background removal: Sonneveld-Visser method, peak threshold: 1.00, intensity
threshold: 10.00
Kα2 removal: intensity ratio: 0.4970
Peak search: second derivative method, σ cut value: 6.00
Profile fitting: Split-type Pearson VII function (fitting to measurement data)

The cyclic conjugated compound in which pyrrole rings are bonded by covalent bonds to be used in the present invention is preferably a porphyrin compound or a phthalocyanine compound, more preferably a phthalocyanine compound from the viewpoint of the spread of a π-electron cloud that becomes a starting point of an interaction. A hydroxygallium phthalocyanine compound is particularly preferred from the viewpoint of an interaction with the resin having a Lewis basic functional group.

Specific examples of the porphyrin compound in the present invention include the following compounds.

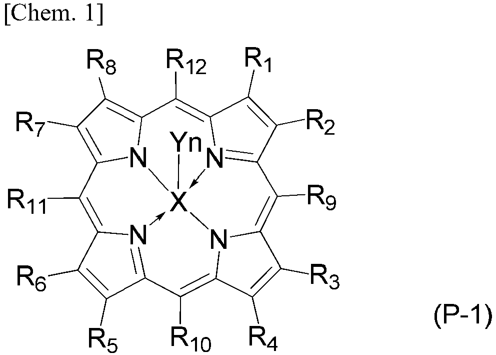

R₁ to R₁₂ each independently represent a hydrogen atom, or an organic group including an aromatic group that may have a substituent or an aliphatic group that may have a substituent.

Specifically, R₁ to R₁₂ preferably each independently represent a hydrogen atom, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sulfonic acid sodium salt group, a 4-cumylphenoxy group, a sulfonic acid group, a phenylthio group, a tert-butyl group, a hydroxy group, a carbonyl group, a methoxy group, an amino group, a sulfo group, or an aldehyde group. Depending on the kinds and number of those groups, the compound may be weakened in crystallinity to be brought close to an amorphous state, or solubility in a solvent may be imparted thereto. For example, when a total of four branched alkyl molecules such as a tert-butyl group, any one of which is used for each benzene ring (e.g., R₁, R₃, R₅, and R₇), are included, the crystallinity tends to be lower and the solubility tends to increase. X represents a metal atom, and specifically, Ga, Ti, V, Al, In, Fe, and Mn are preferred. Y preferably represents a halogen atom, an alkyl group, an aryl group, a carboxy group, an alkoxy group, a hydroxy group, a cyano group, an amino group, or an oxygen atom. "n" represents 1 or 2. When "n" represents 2, a case in which Ys are different from each other is preferred.

Specific examples of the phthalocyanine compound in the present invention include the following compounds.

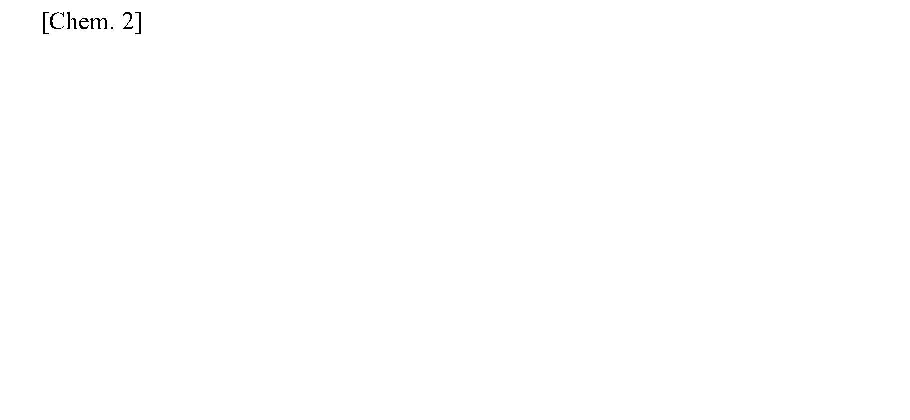

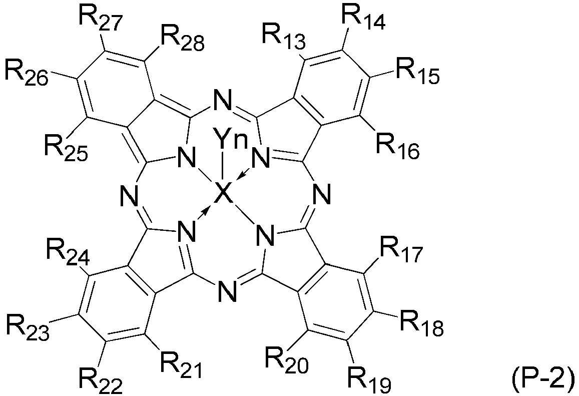

R₁₃ to R₂₈ each independently represent a hydrogen atom, or an organic group including an aromatic group that may have a substituent or an aliphatic group that may have a substituent. Specifically, R₁₃ to R₂₈ each preferably represent a hydrogen atom, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sulfonic acid sodium salt group, a 4-cumylphenoxy group, a sulfonic acid group, a phenylthio group, a tert-butyl group, a hydroxy group, a carbonyl group, a methoxy group, an amino group, a sulfo group, or an aldehyde group. Depending on the kinds and number of those groups, the compound may be weakened in crystallinity to brought close to an amorphous state, or solubility in a solvent may be imparted thereto. For example, when a total of four branched alkyl molecules such as a tert-butyl group, any one of which is used for each benzene ring (e.g., R₁₅, R₁₉, R₂₃, and R₂₇), are included, the crystallinity tends to be lower and the solubility tends to increase. X represents a metal atom, and specifically, Ga, Ti, V, Al, In, Fe, and Mn are preferred. Y preferably represents a halogen atom, an alkyl group, an aryl group, a carboxy group, an alkoxy group, a hydroxy group, a cyano group, an amino group, or an oxygen atom. "n" represents 1 or 2. When "n" represents 2, a case in which Ys are different from each other is preferred.

The charge-transporting layer of the present invention contains the resin having a Lewis basic functional group. Specific examples of the Lewis basic functional group include a hydroxy group, a halogen, a sulfo group, an amino group, a carbonyl group, an ester group, an ether group, a carboxy group, an aldehyde group, a methoxy group, an amide group, a sulfide group, a cyano group, a thienyl group, a pyridyl group, a furyl group, a pyrazolyl group, an imidazolyl group, an oxazolyl group, and a thiazolyl group. Of those, a hydroxy group, a carbonyl group, an ether group, an amino group, an ester group, a pyridyl group, and a thienyl group are each preferred from the viewpoint of an electronic interaction with the cyclic conjugated compound in which pyrrole rings are bonded by conjugated bonds. In particular, the resin having a Lewis basic functional group more preferably has at least one functional group selected from the group consisting of: a hydroxy group; a carbonyl group; an ether group; an amino group; an ester group; a pyridyl group; and a thienyl group, and the resin still more preferably has at least two functional groups selected from the group consisting of: a hydroxy group; a carbonyl group; an ether group; an amino group; an ester group; a pyridyl group; and a thienyl group. Different functional groups may be incorporated into one kind of resin, or two kinds of resins having different functional groups may be mixed. The Lewis basic functional group is preferably incorporated into the repeating structure of the resin. In the present invention, the structure of a chemical substance may be determined by analysis using, for example, nuclear magnetic resonance (NMR) or X-ray photoelectron spectroscopy (XPS).

The weight-average molecular weight of the resin is preferably 10,000 or more from the viewpoint of film formation by an interaction with the cyclic conjugated compound in which pyrrole rings are bonded by conjugated bonds.

Specific examples of the resin having a Lewis basic functional group to be preferably used in the present invention are listed below. The specific examples include polyvinyl butyral, poly(4-vinylpyridine), poly(vinyl chloride), poly(vinylidene fluoride), polyacrylonitrile, poly(vinylidene fluoride-co-hexafluoropropylene), poly(acrylonitrile-co-butadiene), poly(styrene-co-acrylonitrile), polychloroprene, poly(4-chlorostyrene), polymethyl methacrylate, polyvinyl acetate, polyethyleneimine, polyvinyl alcohol, polyacrylic acid, poly(sodium 4-styrenesulfonate), poly(allylamine hydrochloride), sodium polyacrylate, poly(4-styrenesulfonic acid), poly(N-isopropylacrylamide), poly(2-ethyl-2-oxazoline), poly(ethylene-alt-maleic anhydride), poly(2-acrylamido-2-methyl-1-propanesulfonic acid), poly(vinyl sulfate) potassium salt, polyanetholesulfonic acid sodium salt, poly(2-(dimethylamino)ethyl methacrylate) methyl chloride quaternary salt, poly(methyl vinyl ether), poly(2-propylacrylic acid), polyvinylpyrrolidone, polypropylene glycol, poly(propylene carbonate), polyvinyl acetate, poly(tetrahydrofuran), nylon-6, poly(ethylene-co-vinyl acetate), poly(propylene glycol) bis(2-aminopropyl ether), poly(bisphenol A carbonate), poly(1,4-butylene adipate), poly(4-vinylphenol), poly(propylene glycol) monobutyl ether, poly(glycidyl methacrylate), poly(butyl acrylate), poly(ethylene succinate), poly(propylene glycol) methacrylate, nylon 11, nylon 12, poly(2-ethylhexyl acrylate), poly(bisphenol A carbonate), poly(propylene glycol) bis(2-aminopropyl ether), polyetherimide, poly(vinyl formal), poly(vinyl methyl ketone), poly(3-hexylthiophene-2,5-diyl), polyaniline, and a composite of poly(3,4-ethylenedioxythiophene) and polystyrenesulfonic acid (PEDOT:PSS). Of those, polyvinyl butyral, polymethyl methacrylate, poly(3-hexylthiophene-2,5-diyl), polyaniline, polyvinyl acetate, polyvinyl alcohol, polyacrylic acid, poly(2-propylacrylic acid), poly(butyl acrylate), and poly(4-vinylpyridine) are each particularly preferred from the viewpoint of an electronic interaction.

From the viewpoints of an open circuit voltage and durability based on an electronic interaction between the cyclic conjugated compound in which pyrrole rings are bonded by covalent bonds and the resin having a Lewis basic functional group, the content of the resin having a Lewis basic functional group in the charge-transporting layer is preferably 5 to 50 mass%, more preferably 7 to 20 mass% with respect to the content of the cyclic conjugated compound in the charge-transporting layer. In the present invention, the mass ratio of a chemical substance may be determined by, for example, nuclear magnetic resonance (NMR).

In the present invention, the glass transition temperature of the resin having a Lewis basic functional group is preferably 95°C or less. When the glass transition temperature falls within the range, the cyclic conjugated compound in which pyrrole rings are bonded by covalent bonds and the resin having a Lewis basic functional group are easily brought into close contact with each other, and easily interact more electronically. The glass transition temperature may be determined by a differential scanning calorimeter (DSC).

In the present invention, the effect of the present invention is obtained by the presence of the charge-transporting layer between the photoelectric conversion layer and the first electrode. Even when another hole-transporting layer or insulating layer is interposed between the charge-transporting layer and the photoelectric conversion layer, molecules are trapped in the charge-transporting layer, and hence the effects of durability and a high open circuit voltage are obtained. In order to obtain the effect of durability by suppressing the decomposition and migration of the constituent components of the photoelectric conversion layer, the arrangement of the charge-transporting layer in a layer adjacent to the photoelectric conversion layer exhibits the maximum effect. Preferred specific examples of the hole-transporting layer or insulating layer that may be interposed between the charge-transporting layer of the present invention and a charge generation layer include sodium chloride, sodium iodide, potassium iodide, rubidium iodide, cesium acetate, copper(I) bromide, copper(I) iodide, nickel(II) chloride, zinc iodide, germanium dioxide, aluminum acetylacetonate, europium(III) acetylacetonate, 1,8-diaminooctane dihydroiodide, 1,4-butanediamine dihydroiodide, hexylamine hydrobromide, n-octylamine hydrobromide, 2-phenylethylammonium iodide, ethylenediamine dihydroiodide, sodium fluoride, cesium chloride, methylammonium chloride, lead(II) thiocyanate, lead(II) acetate, potassium chloride, niobium(V) fluoride, choline chloride, L-α-phosphatidylcholine, fullerene, (6,6)-phenyl-C61-butyric acid methyl ester [PCBM], iodopentafluorobenzene, F4TCNQ, thiophene, pyridine, pentafluorobenzyl bromide, (3-mercaptopropyl)trimethoxysilane, thiourea, benzylamine, hexamethylenetetramine, N-(3-aminopropyl)-2-pyrrolidinone, theophylline, caffeine, 2-aminoethanesulfonamide hydrochloride, tri-n-octylphosphine oxide, graphene oxide, poly(3-hexylthiophene-2,5-diyl), poly(4-vinylpyridine), polyethylene oxide, polyvinylpyrrolidone, and poly(methyl methacrylate). Of those, sodium chloride, potassium iodide, rubidium iodide, cesium acetate, nickel(II) chloride, aluminum acetylacetonate, n-octylamine hydrobromide, 2-phenylethylammonium iodide, sodium fluoride, cesium chloride, methylammonium chloride, potassium chloride, niobium(V) fluoride, thiophene, pyridine, trimethoxysilane, thiourea, benzylamine, theophylline, poly(4-vinylpyridine), and poly(methyl methacrylate) are particularly preferred.

When the charge-transporting layer is formed by a coating method, a coating liquid for the charge-transporting layer may be prepared by dispersing the crystal of the cyclic conjugated compound in which pyrrole rings are bonded by covalent bonds in a solvent containing the resin having a Lewis basic functional group by various known methods. The application of the coating liquid can form a layer including the crystal of the cyclic conjugated compound in which pyrrole rings are bonded by covalent bonds and the resin having a Lewis basic functional group. Examples of the solvent to be used include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

The thickness of the charge-transporting layer is preferably 1 to 1,000 nm, more preferably 5 to 500 nm, particularly preferably 10 to 200 nm.

As in the above-mentioned mechanism, when the respective constituent elements exert synergistic effects on each other, the effect of the present invention can be achieved.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of an element to be incorporated gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element.

Fig. 1 is a sectional view for schematically illustrating the configuration of a photoelectric conversion element according to one embodiment of the present invention. A photoelectric conversion element 1 of Fig. 1 includes a substrate 2, and a second electrode 3, an electron-transporting layer 4, a photoelectric conversion layer 5, a charge-transporting layer 6, a second charge-transporting layer 7, and a first electrode 8 arranged thereon. A current can be extracted by connecting the first electrode 8 and the second electrode 3 with an external circuit.

The photoelectric conversion layer 5 is excited by light that has entered the layer through the substrate 2, and the second electrode 3 and the electron-transporting layer 4, or the first electrode 8, the second charge-transporting layer 7, and the charge-transporting layer 6 to generate an electron or a hole. That is, the photoelectric conversion layer 5 generates a current between the first electrode 8 and the second electrode 3. The electron-transporting layer 4 is a layer arranged between the photoelectric conversion layer 5 and the second electrode 3, and may not be formed in some cases. The second charge-transporting layer 7 is a layer arranged between the charge-transporting layer 6 and the first electrode 8, and may not be formed in some cases. A form in which the electron-transporting layers 4 and photoelectric conversion layers 5 are laminated is permitted. Such form may also be referred to as "tandem structure."

In Fig. 2, the position of the substrate 2 is different from that in Fig. 1. The first electrode 8, the second charge-transporting layer 7, the charge-transporting layer 6, the photoelectric conversion layer 5, the electron-transporting layer 4, and the second electrode 3 are formed in the stated order on the substrate 2. The effect of the present invention is exhibited even when the position of the substrate 2 is different as described above.

The respective members are described below.

### [Photoelectric Conversion Element]

The photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure, the photoelectric conversion element being characterized by further including, between the photoelectric conversion layer and the first electrode, a charge-transporting layer containing: a crystal of a cyclic conjugated compound in which pyrrole rings are bonded by conjugated bonds, the compound having an axial ligand; and a resin having a Lewis basic functional group. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a crystal having a perovskite structure in its photoelectric conversion layer.

A method of forming each of the layers and electrodes including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer and electrode.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 8 side, an opaque material may be used as the substrate 2, and when light is taken in from the second electrode 3 side, the substrate 2 is formed of a transparent material.

### [Electrode]

A material for the first electrode 8 or the second electrode 3 is not particularly limited, and a material that has hitherto been known may be used. Examples thereof include: metals, such as gold, silver, titanium, and copper; sodium; a sodium-potassium alloy; lithium; a carbon nanotube; magnesium; carbon; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 8 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 8 is on the light incident side, the second electrode 3 and the substrate 2 may be a transparent electrode and a reflective layer, respectively. The transparent electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4] ("n" represents a positive integer).

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule that may have a substituent or of a metal. Specifically, ethylammonium, propylammonium, n-butylammonium, n-hexylammonium, n-octylammonium, 1,6-hexadiammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butyl-benzylammonium, 3-(aminomethyl)piperidinium, and 4-(aminomethyl)piperidinium are preferred.

B in each of the formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred.

The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be incorporated as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

The crystal having a perovskite structure to be used in the present invention is preferably a crystalline semiconductor. The term "crystalline semiconductor" means a semiconductor that enables the measurement of an X-ray scattering intensity distribution to detect a scattering peak. When the crystal having a perovskite structure is the crystalline semiconductor, the mobility of an electron in the crystal having a perovskite structure increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Hole-transporting Layer (Second Charge-transporting Layer)]

In the present invention, the photoelectric conversion element preferably further includes a second charge-transporting layer between the first electrode and the charge-transporting layer from the viewpoint of the compatibility of films.

A material for the second charge-transporting layer 7 is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. In particular, the second charge-transporting layer preferably has an aromatic ring from the viewpoint of the compatibility of a film interface, and more preferably contains Spiro-OMeTA, PTAA, or a phthalocyanine compound.

In addition, the second charge-transporting layer may contain a dopant as an additive in order to improve its charge-transporting ability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in Fig. 1 and Fig. 2.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene compound, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of electron transportation, and hence the photoelectric conversion efficiency increases. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material. The examples are described below.

### [Photoelectric Conversion Apparatus]

A photoelectric conversion apparatus of the present invention includes the photoelectric conversion element of the present invention. The photoelectric conversion apparatus may be formed by using the photoelectric conversion elements of the present invention. When the photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

A moving body of the present invention includes the photoelectric conversion element of the present invention. Fig. 4 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the photoelectric conversion element of the present invention. Fig. 5 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

### [Others]

In addition to the application examples described above, the following application examples may be given: portable devices, such as a calculator, a sensor, and a small solar panel; wearable devices, such as a glasses-type terminal, a watch-type terminal, and a portable medical device; sheet structures supported by frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; forming a photoelectric conversion layer containing a crystal having a perovskite structure between the first electrode and the second electrode; and forming a charge-transporting layer between the photoelectric conversion layer and the first electrode.

The respective steps of the production method are described below.

### (Step of forming First Electrode and Step of forming Second Electrode)

In the step of forming the first electrode and the step of forming the second electrode, appropriate methods may be selected in accordance with a material of the first electrode and a material of the second electrode, respectively. Examples of such methods include, but are not limited to, a sputtering method, a vacuum vapor deposition method, a vapor phase growth method (CVD method), and a spray pyrolysis deposition method (SPD method). Materials of the first electrode and the second electrode are as described above. When one, or each of both, of the first electrode and the second electrode is a transparent electrode, the thickness of the transparent electrode is preferably 0.03 to 3 µm.

When a solar cell is produced, cutting processing may be performed for circuit formation between steps. Examples of the cutting processing include mechanical patterning and laser patterning.

### (Modularization Step)

An element formed up to the electrode may be sealed. A sealing method is, for example, sealing with a resin or sealing with a film. Examples of a material used for the sealing include silazane, silicone rubber, resins each having a siloxane skeleton, and glass.

In addition, hairline treatment may be applied to the surface of the sealed element from the viewpoint of the suppression of adhesion between elements occurring during winding in a roll-to-roll system.

### (Step of forming Photoelectric Conversion Layer)

The step of forming the photoelectric conversion layer may include a step of applying a liquid containing the material of the photoelectric conversion layer as described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or in an inert atmosphere (in a nitrogen or argon atmosphere) in order to remove a solvent or a dispersion medium from the applied liquid containing the material of the photoelectric conversion layer. The temperature of the annealing treatment is preferably 40 to 300°C, more preferably 50 to 150°C. The annealing treatment is preferably performed because materials for forming the respective layers permeate each other at an interface between laminated layers to increase a contact area, and hence a short-circuit current can be increased in some cases.

### (Step of forming Charge-transporting Layer)

As the step of forming the charge-transporting layer, a method including applying a liquid containing the material of the charge-transporting layer as described above is preferred. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. In addition, examples of the step of forming the charge-transporting layer include the following.

That is, examples thereof include: a method including arranging the crystal of the cyclic conjugated compound in which pyrrole rings are bonded by conjugated bonds, the compound having an axial ligand, followed by the application of the resin having a Lewis basic functional group; a method including applying a resin solution in which the resin having a Lewis basic functional group is dissolved, followed by the arrangement of the crystal of the cyclic conjugated compound in which pyrrole rings are bonded by conjugated bonds, the compound having an axial ligand; and a method including applying a solution obtained by dispersing, in the resin solution in which the resin having a Lewis basic functional group is dissolved, the crystal of the cyclic conjugated compound in which pyrrole rings are bonded by conjugated bonds, the compound having an axial ligand.

### Examples

The present invention is described in more detail below by way of Examples and Comparative Examples. The present invention is by no means limited to the following Examples without departing from the gist thereof. In the description of the following Examples, the term "part(s)" is by mass unless otherwise specified.

### (Example 1)

### [Formation of Electron-transporting Layer]

A glass substrate with ITO was washed, and tin(II) oxide whose concentration had been adjusted to 3 mass% was applied thereonto by spin coating. After that, the resultant was heated at 150°C for 30 minutes to form a thin film-shaped electron-transporting layer having a thickness of 15 nm.

### [Formation of Photoelectric Conversion Layer]

22.4 Milligrams of methylammonium bromide, 172 mg of formamidinium iodide, and 576 mg of lead iodide were dissolved in 600 µL of N,N-dimethylformamide and 160 µL of dimethyl sulfoxide, and the solution was stirred for 1 hour (solution 1). Further, 389.7 mg of cesium iodide was dissolved in 1,000 µL of dimethyl sulfoxide, and the solution was stirred for 1 hour (solution 2). After that, 40 µL of the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.96}Pb(I_{0.95}Br_{0.05})₃ and having a thickness of 400 nm.

### [Formation of Charge-transporting Layer]

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71 mass%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71 mass%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine particle having a water content of 1.0 mass% or less was obtained.

### Step (3)

5 Parts of the hydroxygallium phthalocyanine particle was mixed with 5 parts of dimethylformamide, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) having sealed therein 5 parts of glass beads, followed by filtration and drying to provide a particle 1.

### <X-ray Diffraction Measurement of Particle 1>

Measurement was performed in accordance with the method described above. The full width at half maximum of each of a first peak and a second peak was 1.0° or less.

### <Production of Resin Solution 1>

1.0 Gram of polyvinyl butyral (product name: BM-2, manufactured by Sekisui Chemical Co., Ltd., glass transition temperature: 71°C) was dissolved in 19 g of 2-propanol by stirring for 24 hours to provide a resin solution 1.

0.1 Gram of the particle 1 and 0.01 g of a calixarene compound (Japanese Patent Laid-Open No. 2003-207913) were mixed with 10.6 g of 2-propanol, and 11 g of zirconia beads were loaded into the mixture, followed by paint shaker dispersion (manufactured by Toyo Seiki Co., Ltd.) for 6 hours. After that, 0.2 g of the resin solution 1 was added thereto, and paint shaker dispersion was performed again for 6 hours to prepare a coating liquid for a charge-transporting layer. The coating liquid for a charge-transporting layer was applied onto the photoelectric conversion layer by spin coating to form a charge-transporting layer having a thickness of 180 nm.

### [Formation of Second Charge-transporting Layer]

0.15 Gram of Spiro-OMeTAD serving as a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of t-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a coating liquid for a second charge-transporting layer. The solution was applied onto the above-mentioned charge-transporting layer by a spin coating method to form a second charge-transporting layer having a thickness of 200 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### [Analysis of Amount of Compound]

The electrode surface of the photoelectric conversion element was peeled off to expose the surface of the charge-transporting layer. The surface of the charge-transporting layer was wiped with a cotton swab or the like with a solvent, dissolved in deuterated sulfuric acid, and subjected to ¹H-NMR measurement (apparatus: AVANCE III 500 manufactured by BRUKER). In addition, the mass and structure analysis of the peeled-off charge-transporting layer components was performed by elemental analysis, such as GPC and MALDI-TOF-MS, IR, gas chromatography, XPS, and EDX, to recognize the presence of a compound.

Phthalocyanine was recovered from the charge-transporting layer of the photoelectric conversion element, and was subjected to XRD measurement in accordance with the method described above. Thus, it was recognized that the full width at half maximum of each of its first peak and second peak was 1.0° or less.

The thickness of the charge-transporting layer was determined with a cross-sectional SEM (apparatus: SmartSEM manufactured by Carl Zeiss Co., Ltd.) after the cutting of the photoelectric conversion element and the fixing of the sample to a tilted sample stage.

### (Example 2)

A photoelectric conversion element is produced in the same manner as in Example 1 except that the cyclic conjugated compound in which pyrrole rings are bonded by conjugated bonds is changed to titanyl phthalocyanine.

### (Example 3)

A photoelectric conversion element is produced in the same manner as in Example 1 except that the cyclic conjugated compound in which pyrrole rings are bonded by conjugated bonds is changed to chlorogallium phthalocyanine.

### (Example 4)

Layers up to a photoelectric conversion layer are formed in the same manner as in Example 1. After that, a liquid prepared by dissolving 2.49 mg of 2-phenethylethylamine hydroiodide in 1 mL of 2-propanol is applied onto the photoelectric conversion layer by spin coating to form a layer having a thickness of 20 nm. After that, a charge-transporting layer, a second charge-transporting layer, and a first electrode are formed in the same manner as in Example 1.

### (Example 5)

A photoelectric conversion element is produced in the same manner as in Example 1 except that the second charge-transporting layer is not formed.

### (Example 6)

A photoelectric conversion element is produced in the same manner as in Example 1 except that 0.2 g of the particle 1 is used for the preparation of the coating liquid for a charge-transporting layer.

### (Example 7)

A photoelectric conversion element is produced in the same manner as in Example 1 except that 0.14 g of the resin solution 1 is used for the preparation of the coating liquid for a charge-transporting layer.

### (Example 8)

A photoelectric conversion element is produced in the same manner as in Example 1 except that 0.40 g of the resin solution 1 is used for the preparation of the coating liquid for a charge-transporting layer.

### (Example 9)

A photoelectric conversion element is produced in the same manner as in Example 1 except that, in the preparation of the coating liquid for a charge-transporting layer, the resin having a Lewis basic functional group is changed to poly(3-hexylthiophene-2,5-diyl) P3HT (weight-average molecular weight: 50,000 to 100,000, manufactured by Sigma-Aldrich Co. LLC), the solvent to be used is changed from 2-propanol to monochlorobenzene to prepare a resin solution, and 1 g of the resin solution is used when the coating liquid for a charge-transporting layer is prepared.

### (Example 10)

A photoelectric conversion element is produced in the same manner as in Example 1 except that, in the preparation of the coating liquid for a charge-transporting layer, the resin having a Lewis basic functional group is changed to poly(3-hexylthiophene-2,5-diyl) P3HT (weight-average molecular weight: 50,000 to 100,000, manufactured by Sigma-Aldrich Co. LLC), the solvent to be used is changed from 2-propanol to monochlorobenzene to prepare a resin solution, and 2 g of the resin solution is used when the coating liquid for a charge-transporting layer is prepared.

### (Example 11)

A photoelectric conversion element is produced in the same manner as in Example 1 except that, in the preparation of the coating liquid for a charge-transporting layer, the resin having a Lewis basic functional group is changed to poly(3-hexylthiophene-2,5-diyl) P3HT (weight-average molecular weight: 50,000 to 100,000, manufactured by Sigma-Aldrich Co. LLC), the solvent to be used is changed from 2-propanol to monochlorobenzene to prepare a resin solution, and 0.24 g of the resin solution is used when the coating liquid for a charge-transporting layer is prepared.

### (Example 12)

A photoelectric conversion element is produced in the same manner as in Example 1 except that, in the preparation of the coating liquid for a charge-transporting layer, the resin having a Lewis basic functional group is changed to polyaniline (weight-average molecular weight: 65,000, manufactured by Sigma-Aldrich Co. LLC), and the solvent to be used is changed from 2-propanol to N-methyl-2-pyrrolidone to prepare a resin solution.

### (Example 13)

A photoelectric conversion element is produced in the same manner as in Example 1 except that, in the preparation of the coating liquid for a charge-transporting layer, the resin having a Lewis basic functional group is changed to a polymethyl methacrylate resin PMMA (manufactured by Sigma-Aldrich Co. LLC, glass transition temperature: 70°C), and the solvent to be used is changed from 2-propanol to monochlorobenzene to prepare a resin solution.

### (Example 14)

A photoelectric conversion element is produced in the same manner as in Example 1 except that, in the preparation of the coating liquid for a charge-transporting layer, the resin having a Lewis basic functional group is changed to poly(4-vinylpyridine) (weight-average molecular weight: 60,000, manufactured by Sigma-Aldrich Co. LLC, glass transition temperature: 137°C).

### (Example 15)

A photoelectric conversion element is produced in the same manner as in Example 1 except that, in the preparation of the coating liquid for a charge-transporting layer, the resin having a Lewis basic functional group is changed to polyacrylonitrile (weight-average molecular weight: 150,000, manufactured by Sigma-Aldrich Co. LLC, glass transition temperature: 85°C), and the solvent to be used is changed from 2-propanol to dimethylformamide to prepare a resin solution.

### (Example 16)

A photoelectric conversion element is produced in the same manner as in Example 1 except that the cyclic conjugated compound in which pyrrole rings are bonded by conjugated bonds is changed to 5,10,15,20-tetraphenyl-21H,23H-porphine iron chloride.

### (Example 17)

A photoelectric conversion element is produced in the same manner as in Example 1 except that the cyclic conjugated compound in which pyrrole rings are bonded by conjugated bonds is changed to vanadyl phthalocyanine.

### (Example 18)

A photoelectric conversion element is produced in the same manner as in Example 1 except that the cyclic conjugated compound in which pyrrole rings are bonded by conjugated bonds is changed to chloroindium phthalocyanine.

### (Example 19)

A photoelectric conversion element is produced in the same manner as in Example 1 except that the cyclic conjugated compound in which pyrrole rings are bonded by conjugated bonds is changed to chloroaluminum phthalocyanine.

### (Example 20)

A photoelectric conversion element is produced in the same manner as in Example 1 except that the cyclic conjugated compound in which pyrrole rings are bonded by conjugated bonds is changed to 5,10,15,20-tetraphenyl-21H,23H-porphine manganese chloride.

### (Example 21)

A photoelectric conversion element is produced in the same manner as in Example 1 except that, in the preparation of the coating liquid for a charge-transporting layer, the resin having a Lewis basic functional group is changed to a polymethyl methacrylate resin (PMMA, manufactured by Sigma-Aldrich Co. LLC, glass transition temperature: 100°C), and the solvent to be used is changed from 2-propanol to monochlorobenzene to prepare a resin solution.

### (Example 22)

A photoelectric conversion element is produced in the same manner as in Example 1 except that, in the preparation of the coating liquid for a charge-transporting layer, the resin having a Lewis basic functional group is changed to a polyvinyl acetal resin (product name: BX-1, manufactured by Sekisui Chemical Co., Ltd., glass transition temperature: 95°C).

### (Example 23)

A photoelectric conversion element is produced in the same manner as in Example 1 except that, in the preparation of the coating liquid for a charge-transporting layer, the resin having a Lewis basic functional group is changed to a polyvinyl acetal resin (product name: KS-10, manufactured by Sekisui Chemical Co., Ltd., glass transition temperature: 105°C), and the solvent to be used is changed from 2-propanol to ethanol to prepare a resin solution.

### (Comparative Example 1)

A photoelectric conversion element was produced in the same manner as in Example 1 except that, in the method of producing the resin solution 1, the resin having a Lewis basic functional group was not used.

### (Comparative Example 2)

A photoelectric conversion element is produced in the same manner as in Comparative Example 1 except that the cyclic conjugated compound in which pyrrole rings are bonded by conjugated bonds is changed to copper phthalocyanine.

### (Comparative Example 3)

A photoelectric conversion element is produced in the same manner as in Example 1 except that the cyclic conjugated compound in which pyrrole rings are bonded by conjugated bonds is changed to 2,9,16,23-tetra-tert-butyl-29H,31H-chloroaluminum phthalocyanine, and in the preparation of the coating liquid for a charge-transporting layer, 2-propanol is changed to chloroform.

### (Comparative Example 4)

A photoelectric conversion element is produced in the same manner as in Example 1 except that, in the preparation of the coating liquid for a charge-transporting layer, the resin is changed to poly(9,9-dioctylfluorenyl-2,7-diyl) (weight-average molecular weight: 50,000 to 150,000, manufactured by Sigma-Aldrich Co. LLC), and the solvent to be used is changed from 2-propanol to xylene.

### (Comparative Example 5)

A photoelectric conversion element is produced in the same manner as in Example 1 except that the cyclic conjugated compound in which pyrrole rings are bonded by conjugated bonds is changed to copper phthalocyanine.

### (Comparative Example 6)

A photoelectric conversion element is produced in the same manner as in Example 1 except that the cyclic conjugated compound in which pyrrole rings are bonded by conjugated bonds is changed to zinc phthalocyanine.

### (Comparative Example 7)

A photoelectric conversion element is produced in the same manner as in Example 1 except that, in the preparation of the coating liquid for a charge-transporting layer, the particle 1 is changed to Spiro-OMeTAD, the resin having a Lewis basic functional group is changed to a polymethyl methacrylate resin (PMMA, manufactured by Sigma-Aldrich Co. LLC, glass transition temperature: 100°C), and the solvent to be used is changed from 2-propanol to monochlorobenzene to prepare a resin solution.

### [Evaluation]

### (Open Circuit Voltage Evaluation)

A power source (manufactured by Keithley Instruments, Model 236) was connected between the electrodes of the photoelectric conversion element of Example 1, and constant light was applied with a solar simulator (manufactured by Yamashita Denso Corporation) at an intensity of 100 mW/cm², followed by the measurement of a current and a voltage to be generated. Thus, an open circuit voltage was evaluated. The result is shown in Table 3. Examples 2 to 23 and Comparative Examples 1 to 7 are each also evaluated for its open circuit voltage in the same manner as in Example 1. The results are shown in Table 3.

### (Evaluation of Durability)

The durability of the photoelectric conversion element of Example 1 was evaluated by continuously applying light of 10,000 Lx thereto with a white LED and measuring the open circuit voltage thereof after 30 days. The evaluation was made by the maintenance rate of the open circuit voltage after 30 days with respect to the initial open circuit voltage thereof. The result is shown in Table 3. Examples 2 to 23 and Comparative Examples 1 to 7 are also evaluated in the same manner as in Example 1, and the durability of each of these Examples and Comparative Examples is evaluated as the maintenance rate of the open circuit voltage thereof after 30 days with respect to the initial open circuit voltage thereof. The results are shown in Table 3.

### (Evaluation of X-ray Diffraction Measurement)

As a result of the X-ray diffraction measurement of a cyclic conjugated compound, a case in which the full width at half maximum of each of the maximum peak and the next largest peak was 1.0° or less was indicated by the symbol "A", and a case in which the condition was not satisfied was indicated by the symbol "B". In the present invention, in the case of the symbol "A", it was determined that the cyclic conjugated compound was crystalline, and in the case of the symbol "B", it was determined that the cyclic conjugated compound was not crystalline. The results are shown in Table 3.

### [Table 2]

**Table 2**

| Example | Cyclic conjugated compound | | Resin | | | | Mass of resin to conjugated compound (%) |
|---|---|---|---|---|---|---|---|
| | Kind | Part(s) by mass | Lewis basic functional group | | | Part(s) by mass | |
| Example 1 | Hydroxygallium phthalocyanine | 0.1 | Ester bond | Hydroxy group | Ether bond | 0.010 | 10 |
| Example 2 | Titanyl phthalocyanine | 0.1 | Ester bond | Hydroxy group | Ether bond | 0.010 | 10 |
| Example 3 | Chlorogallium phthalocyanine | 0.1 | Ester bond | Hydroxy group | Ether bond | 0.010 | 10 |
| Example 4 | Hydroxygallium phthalocyanine | 0.1 | Ester bond | Hydroxy group | Ether bond | 0.010 | 10 |
| Example 5 | Hydroxygallium phthalocyanine | 0.1 | Ester bond | Hydroxy group | Ether bond | 0.010 | 10 |
| Example 6 | Hydroxygallium phthalocyanine | 0.2 | Ester bond | Hydroxy group | Ether bond | 0.010 | 5 |
| Example 7 | Hydroxygallium phthalocyanine | 0.1 | Ester bond | Hydroxy group | Ether bond | 0.007 | 7 |
| Example 8 | Hydroxygallium phthalocyanine | 0.1 | Ester bond | Hydroxy group | Ether bond | 0.020 | 20 |
| Example 9 | Hydroxygallium phthalocyanine | 0.1 | Thienyl group | - | - | 0.050 | 50 |
| Example 10 | Hydroxygallium phthalocyanine | 0.1 | Thienyl group | - | - | 0.100 | 100 |
| Example 11 | Hydroxygallium phthalocyanine | 0.1 | Thienyl group | - | - | 0.012 | 12 |
| Example 12 | Hydroxygallium phthalocyanine | 0.1 | Amino group | - | - | 0.010 | 10 |
| Example 13 | Hydroxygallium phthalocyanine | 0.1 | Ester bond | - | - | 0.010 | 10 |
| Example 14 | Hydroxygallium phthalocyanine | 0.1 | Pyridine | - | - | 0.010 | 10 |
| Example 15 | Hydroxygallium phthalocyanine | 0.1 | Cyano group | - | - | 0.010 | 10 |
| Example 16 | 5,10,15,20-Tetraphenyl-21H,23H-porphine iron chloride | 0.1 | Ester bond | Hydroxy group | Ether bond | 0.010 | 10 |
| Example 17 | Vanadyl phthalocyanine | 0.1 | Ester bond | Hydroxy group | Ether bond | 0.010 | 10 |
| Example 18 | Chloroindium phthalocyanine | 0.1 | Ester bond | Hydroxy group | Ether bond | 0.010 | 10 |
| Example 19 | Chloroaluminum phthalocyanine | 0.1 | Ester bond | Hydroxy group | Ether bond | 0.010 | 10 |
| Example 20 | 5,10,15,20-Tetraphenyl-21H,23H-porphine manganese chloride | 0.1 | Ester bond | Hydroxy group | Ether bond | 0.010 | 10 |
| Example 21 | Hydroxygallium phthalocyanine | 0.1 | Ester bond | - | - | 0.010 | 10 |
| Example 22 | Hydroxygallium phthalocyanine | 0.1 | Ester bond | Hydroxy group | Ether bond | 0.010 | 10 |
| Example 23 | Hydroxygallium phthalocyanine | 0.1 | Ester bond | Hydroxy group | Ether bond | 0.010 | 10 |
| Comparative Example 1 | Hydroxygallium phthalocyanine | 0.1 | - | - | - | - | 0 |
| Comparative Example 2 | Copper phthalocyanine | 0.1 | - | - | - | - | 0 |
| Comparative Example 3 | 2,9,16,23-Tetra-tert-butyl-29H,31H-chloroaluminum phthalocyanine | 0.1 | Ester bond | Hydroxy group | Ether bond | 0.010 | 10 |
| Comparative Example 4 | Hydroxygallium phthalocyanine | 0.1 | - | - | - | 0.010 | 10 |
| Comparative Example 5 | Copper phthalocyanine | 0.1 | Ester bond | Hydroxy group | Ether bond | 0.010 | 10 |
| Comparative Example 6 | Zinc phthalocyanine | 0.1 | Ester bond | Hydroxy group | Ether bond | 0.010 | 10 |
| Comparative Example 7 | Spiro-OMeTAD | 0.1 | Ester bond | - | - | 0.010 | 10 |

### [Table 3]

**Table 3**

| Example | Open circuit voltage (V) | Maintenance rate (%) | X-ray diffraction measurement |
|---|---|---|---|
| Example 1 | 1.16 | 96 | A |
| Example 2 | 1.09 | 91 | A |
| Example 3 | 1.10 | 90 | A |
| Example 4 | 1.15 | 97 | A |
| Example 5 | 1.08 | 91 | A |
| Example 6 | 1.10 | 95 | A |
| Example 7 | 1.15 | 97 | A |
| Example 8 | 1.16 | 97 | A |
| Example 9 | 1.10 | 92 | A |
| Example 10 | 1.11 | 89 | A |
| Example 11 | 1.08 | 93 | A |
| Example 12 | 1.09 | 92 | A |
| Example 13 | 1.10 | 93 | A |
| Example 14 | 1.10 | 93 | A |
| Example 15 | 1.02 | 92 | A |
| Example 16 | 1.10 | 88 | A |
| Example 17 | 1.09 | 90 | A |
| Example 18 | 1.08 | 91 | A |
| Example 19 | 1.09 | 90 | A |
| Example 20 | 1.10 | 88 | A |
| Example 21 | 1.07 | 91 | A |
| Example 22 | 1.15 | 95 | A |
| Example 23 | 1.10 | 95 | A |
| Comparative Example 1 | 0.95 | 88 | A |
| Comparative Example 2 | 1.09 | 70 | A |
| Comparative Example 3 | 0.95 | 75 | B |
| Comparative Example 4 | 0.96 | 90 | A |
| Comparative Example 5 | 1.10 | 77 | A |
| Comparative Example 6 | 1.09 | 75 | A |
| Comparative Example 7 | 0.95 | 70 | B |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, and Japanese Patent Application No. 2024-086009 filed on May 28, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 second charge-transporting layer
8 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element further comprises, between the photoelectric conversion layer and the first electrode, a charge-transporting layer containing: a crystal of a cyclic conjugated compound in which pyrrole rings are bonded by conjugated bonds, the compound having an axial ligand; and a resin having a Lewis basic functional group.

2. The photoelectric conversion element according to claim 1, wherein the photoelectric conversion element further comprises a second charge-transporting layer between the first electrode and the charge-transporting layer.

3. The photoelectric conversion element according to claim 1 or 2, wherein the resin having a Lewis basic functional group has at least one functional group selected from the group consisting of: a hydroxy group; a carbonyl group; an ether group; an amino group; an ester group; a pyridyl group; and a thienyl group.

4. The photoelectric conversion element according to claim 3, wherein the resin having a Lewis basic functional group has at least two functional groups selected from the group consisting of: a hydroxy group; a carbonyl group; an ether group; an amino group; an ester group; a pyridyl group; and a thienyl group.

5. The photoelectric conversion element according to any one of claims 1 to 4, wherein a content of the resin having a Lewis basic functional group in the charge-transporting layer is 5 to 50 mass% with respect to a content of the cyclic conjugated compound in the charge-transporting layer.

6. The photoelectric conversion element according to claim 5, wherein the content of the resin having a Lewis basic functional group in the charge-transporting layer is 7 to 20 mass% with respect to the content of the cyclic conjugated compound in the charge-transporting layer.

7. The photoelectric conversion element according to any one of claims 1 to 6, wherein the cyclic conjugated compound has at least one central element selected from the group consisting of: Ga; Ti; V; Al; In; Fe; and Mn.

8. The photoelectric conversion element according to any one of claims 1 to 7, wherein the number of the axial ligands of the cyclic conjugated compound is one.

9. The photoelectric conversion element according to any one of claims 1 to 8, wherein the axial ligand is at least one selected from the group consisting of: OH; Cl; and O.

10. The photoelectric conversion element according to any one of claims 1 to 9, wherein the cyclic conjugated compound is a phthalocyanine compound.

11. The photoelectric conversion element according to claim 10, wherein the phthalocyanine compound is a hydroxygallium phthalocyanine compound.

12. The photoelectric conversion element according to any one of claims 1 to 11, wherein the resin having a Lewis basic functional group has a glass transition temperature of 95°C or less.

13. A photoelectric conversion apparatus comprising the photoelectric conversion element of any one of claims 1 to 12.
